Europäisches Patentamt

(19) European Patent Office

Office européen des brevets

(11) Numéro de publication: **0 017 595**
**B1**

(12) **FASCICULE DE BREVET EUROPEEN**

(45) Date de publication du fascicule du brevet: **12.01.83**

(51) Int. Cl.³: **A 61 K 7/48**

(21) Numéro de dépôt: **80400469.5**

(22) Date de dépôt: **09.04.80**

(54) Utilisation de produits cosmétiques à base de substances foetales congelées par application directe sur la peau.

(30) Priorité: **10.04.79 FR 7909003**

(43) Date de publication de la demande:
**15.10.80 Bulletin 80/21**

(45) Mention de la délivrance du brevet:
**12.01.83 Bulletin 83/2**

(84) Etats contractants désignés:
**AT BE CH DE GB IT LI LU NL SE**

(56) Documents cités:
**FR - A - 2 128 265**
**FR - A - 2 147 334**
**FR - A - 2 192 800**
**FR - A - 2 194 418**
**FR - A - 2 336 121**

**Le dossier contient des informations techniques présentées postérieurement au dépôt de la demande et ne figurant pas dans le présent fascicule.**

(73) Titulaire: **Etablissement TROCO**
**Aeulestrasse 38**
**Vaduz (LI)**

(72) Inventeur: **Bontemps, Raymond**
**5, rue Edouard Detaille**
**F-75017 Paris (FR)**

(74) Mandataire: **Blétry, Robert et al,**
**OFFICE BLETRY 2, Boulevard de Strasbourg**
**F-75010 Paris (FR)**

Utilisation de produits cosmétiques à base de substances foetales congelées par application directé sur la peau

La présente invention s'applique essentiellement à des produits cosmétiques se présentant sous la forme de blocs congelés à base de substances foetales pures choisies parmi le foie, le placenta, le mésenchyme, le thymus, l'amnios, le liquide allantoïde, le cartilage de Meckel, la gelée de Wharton et les mélanges de ces substances animales.

Certains de ces produits d'origine foetale sont en fait des biogènes, c'est-à-dire des cellules aspécifiques (à base d'ADN et d'ARN) qui sont destinées à créer la matière vivante.

Des compositions ou préparations cosmétiques congelées sont déjà connues par les demandes de brevets français nº 2.147.334 (L'OREAL), nº 2.128.265 et nº 2.192.800 (SADA) ainsi que par la demande française nº 2.336.121 au nom du présent demandeur.

La demande française nº 2.147.334 (L'OREAL) décrit un procédé de conservation et de conditionnement de compositions cosmétiques se présentant à la température ordinaire sous la forme d'une solution aqueuse ou hydroalcoolique et comportant un ou plusieurs constituants qui, en solution, sont instables ou incompatibles entre eux à la température ordinaire. Conformément à ce procédé, on prépare la composition en mélangeant les différents constituants; on abaisse la température de la composition au-dessous de son point de congélation immédiatement après le mélange; et on conserve le solide obtenu dans un réceptacle approprié à une température inférieure audit point de congélation. La composition peut être congelée de façon à obtenir un solide de dimensions relativement importantes, et qui peut être par la suite divisé en plusieurs parties avant son conditionnement. Elle peut être ainsi conditionnée sous la forme de blocs contenant la quantité requise pour une application et distribués dans des récipients quelconques tels que sachets en matière plastique ou tout autre emballage analogue.

La demande française nº 2.128.265 (SADA) se rapporte à un procédé de production, conservation et distribution de préparations constituées de substances naturelles à destination cosmétique, suivant lequel on surgèle la substance organique naturelle, préalablement mélangée avec les additifs habituels des compositions cosmétiques et de type naturel; on divise ces préparations en doses unitaires destinées à une seule application; on conditionne ces doses sous vide en emballages unitaires (enveloppes ou feuilles d'aluminium thermosoudables); on conserve et on distribue ces produits à l'état surgelé.

La demande française nº 2.192.800 (SADA) concerne, comme le précédent document SADA, la distribution de produits cosmétiques dans de petites enveloppes individuelles à l'état ultrarefroidi.

L'utilisation à des fins cosmétiques des substances prélevées sur les poches foetales d'animaux est déjà connue et, ainsi que le montre le brevet français nº 2.336.121 au nom de Monsieur Raymond BONTEMPS, il est avantageux de conserver ces substances à l'état frais, de façon à pouvoir les appliquer exemptes de tout excipient et de tout conservateur. A cet effet, ces substances, prélevées sur l'animal, réduites en purée et éventuellement associées, sont conditionnées à une température de l'ordre de −80°C.

Conformément aux indications des demandes de brevets précitées, à l'utilisation, les substances cosmétiques sont toujours ramenées à la température ambiante; ainsi, selon la demande de brevet précitée au nom de Monsieur BONTEMPS, au moment de l'emploi, il est prévu de dégeler les substances foetales et d'y incorporer éventuellement un parfum et/ou un liquide isotonique comme le sérum physiologique et/ou une substance pénétrant dans la peau, dans la proportion de 1 à 30%.

Ces préparations cosmétiques d'origine foetale, qui sont utilisées comme masques ou produits de massage, donnent toute satisfaction.

La présente invention permet toutefois de simplifier encore davantage les conditions d'utilisation de ces produits cosmétiques d'origine foetale, tout en améliorant encore leur efficacité.

La présente invention a en effet pour objet une utilisation des produits cosmétiques du type défini plus haut, suivant laquelle lesdits produits sont appliqués directement sur la peau dans leur état congelé sans décongélation préalable à une température d'environ −10°C.

Lors de l'application des produits cosmétiques se présentant sous la forme de blocs ou pains congelés permettant chacun des applications multiples, l'action vasoconstrictrice du froid viendra s'ajouter à l'action proprement dite de ces substances cosmétiques, assurant ainsi leur pénétration rapide dans l'épiderme.

Il sera très aisé de conserver à basse température ces blocs ou pains dans leurs moules de congélation, réalisés par exemple en métal ou en matière plastique. On pourra également les conserver démoulés, l'important étant que la chaîne du froid ne soit pas rompue de la fabrication à l'utilisation.

En outre, conformément à la présente invention, on prévoira également des moyens pour supporter les blocs congelés afin de faciliter leur utilisation.

Par exemple, les blocs, parallélépipédiques, tronconiques ou de toute autre forme, seront moulés autour de supports (bâtonnets par exemple) en bois, matière plastique ou similaire, dont les extrémités, dépassant des blocs, en permettront une manipulation aisée.

De préférence, chacun de ces supports sera rendu solidaire d'un couvercle du moule. L'utilisateur pourra ainsi retirer le bloc congelé de son moule sans qu'il y ait de contact avec la substance cosmétique.

A titre de variante, on pourra présenter le bloc congelé dans un étui cylindrique du type de ceux des tubes de rouge à lèvres ou de fards, ou encore de désodorisants solides, l'utilisateur faisant sortir hors de l'étui l'extrémité supérieure du bloc (stick) à chacune des applications.

Le procédé de conditionnement de ces blocs ou pains consiste à couler ou comprimer dans des moules appropriés la substance cosmétique choisie ou l'association choisie de substances cosmétiques, et à réfrigérer rapidement et intensivement jusqu'à congélation complète, soit entre −25°C et −80°C (neige carbonique).

Les blocs de substances cosmétiques ainsi formés peuvent être conservés indéfiniment à leur température de préparation; ramenés à −10°C environ en vue de leur utilisation, ils pourront se conserver encore quelque temps (à peu près 1 mois) à cette température, sans perdre de leur efficacité.

Ainsi l'utilisatrice pourra aisément conserver les blocs cosmétiques dans son congélateur ou dans le compartiment de son réfrigérateur où se forme le glace.

L'application d'un bloc gelé sur le visage conduit à développer sur la peau un film composé en générale exclusivement de substance active, le bloc fondant en surface lorsqu'il est à la température ambiante. La substance active présente à ce moment une efficacité maximale en raison d'une part de l'action directe des substances conservées à l'état frais, et d'autre part de sa pénétration rapide dans l'épiderme par effet vasoconstricteur, la contraction des fibres musculaires des vaisseaux de l'épiderme assurant un "emprisonnement" des fines particules de substance cosmétique.

Après chaque application sur la peau, l'utilisatrice replace le bloc dans son congélateur jusqu'à épuisement de ce bloc. Il n'y a jamais de manipulation intermédiaire (contact des produits cosmétiques avec les mains); le bloc congelé ne perd jamais ainsi son efficacité.

Cette technique d'application est particulièrement avantageuse pour les substances cosmétiques d'origine foetale pour lesquelles il est important qu'elles conservent exactement toutes les propriétés qu'elles possédaient au moment de leur prélèvement.

On donnera ci-'après deux exemples de préparation de pains cosmétiques selon l'invention; comme substance active, on préfère le liquide allantoïde, éventuellement associé à l'amnios, au placenta et/ou au foie.

### Exemple I

On effectue un prélèvement *stérile* du liquide allantoïde pur (produit nourricier du foetus) dans une poche foetale bovine, à l'aide d'une pompe à circuit fermé, liquide qui sera réparti immédiatement dans des moules, stérilisés eux aussi auparavant.

Cette opération sera faite sous flux laminaire (c'est-à-dire surpression d'air dans la pièce), dans un délai rapide après l'abattage de la bête (en moyenne trois heures).

Les moules remplis de substance sont abaissés très rapidement à une température de −80°C, afin de préserver l'intégralité de toute la formule biochimique et des acides aminés qui y sont contenus.

### Exemple II

Une préparation peut également être faite avec, par exemple, 300 g de placenta, 200 g de foie (pour la pigmentation de la peau), 500 g d'allantoïdes et 300 g de soluté physiologique. Ces substances seront passées au robot de coupe (mixer) et filtrées, puis réparties dans les moules et congelées toujours dans un délai ne dépassant pas trois heures après le prélèvement, dans les mêmes conditions que précédemment.

Sur le dessin ci-annexé, on a représenté trois exemples possibles de moules pour les pains cosmétiques selon l'invention.

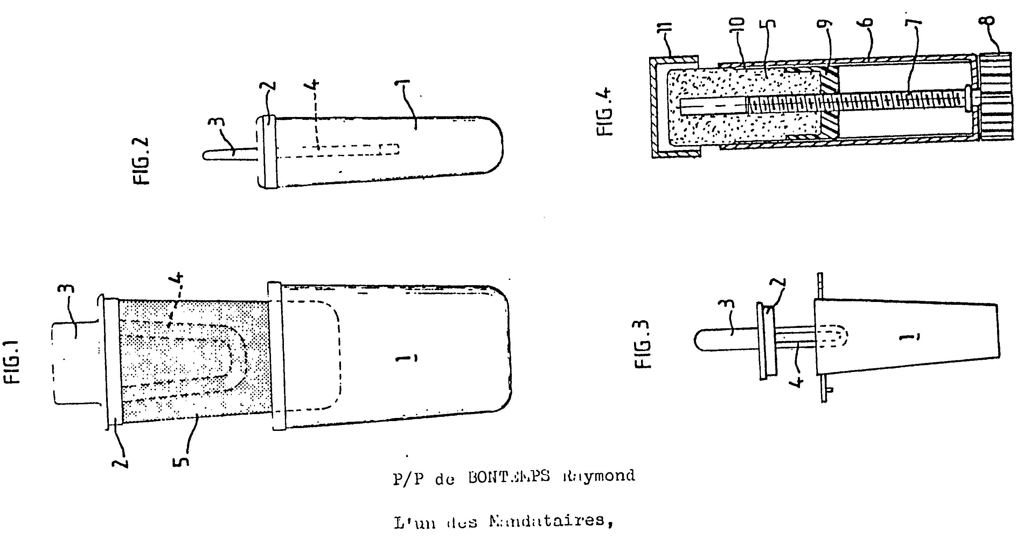

Les figures 1 et 2 représentent un moule sensiblement parallélépipédique, respectivement en élévation et de profil, la figure 3 montre un moule tronconique et la figure 4 un réceptacle pour un "stick".

Les moules 1 (figures 1, 2 et 3) sont susceptibles d'être fermés par un couvercle 2 portant extérieurement une languette 3 en permettant la préhension et intérieurement un support 4 du bloc cosmétique congelé 5.

Dans le cas du "stick", (figure 4), le bloc congelé 5 est susceptible de progresser en dehors de son réceptable cylindrique 6 au centre duquel est disposée une vis 7 manoeuvrable par un bouton 8 situé à la base du cylindre 6. Le bloc 5 est maintenu à sa partie inférieure par une embase 9 et il est guidé dans son déplacement vertical par des nervures intérieures verticales 10 du cylindre 6. L'ensemble est protégé par un capuchon 11.

### Revendication

Utilisation de produits cosmétiques se présentant sous la forme de blocs congelés à base de substances foetales pures choisies parmi le foie, le placenta, le mésenchyme, le thymus, l'amnios, le liquide allantoïde, le cartilage de Meckel, la gelée de Wharton et les mélanges de ces substances, caractérisée en ce que ces produits cosmétiques sont appliqués directement sur la peau dans leur état congelé sans décongélation préalable à une température d'environ —10°C.

**Patentanspruch**

Verwendung von kosmetischen Produkten, die in Form von gefrorenen Blöcken auf der Basis von reinen fötalen Substanzen, ausgewählt aus der Leber, der Placenta, dem Mesenchym, dem Thymus, dem Amnion, der allantoiden Flüssigkeit, dem Meckel-Knorpel, der Wharton-Sulze oder Mischungen dieser Substanzen, vorliegen, dadurch gekennzeichnet, daß die kosmetischen Produkte in ihrem gefrorenen Zustand ohne vorheriges Auftauen bei einer Temperatur von ungefähr —10°C direkt auf die Haut aufgebracht werden.

**Claim**

Use of cosmetic products in the form of frozen blocks bases on pure foetal substances chosen from the liver, placenta, mesenchyma, thymus, amnion, allantoic liquid, Meckel's cartilage, Wharton's jelly and mixtures of these substances, characterized in that these cosmetic products are applied directly to the skin in their frozen state, without prior thawing, at a temperature of about —10°C.

**0017 595**

FIG.1

3

2

5

4

1

FIG.2

3

2

4

1

P/P de BONT.h.PS Raymond

L'un des Mandataires,

FIG.3

3

2

4

1

FIG.4

11

10

5

9

6

7

8

1